# EUROPEAN PATENT APPLICATION

(11) **EP 4 331 470 A1**
(43) Date of publication of application: **06.03.2024**
(21) Application number: 22382810.4
(22) Date of filing: 30.08.2022
(51) Int. Cl.: A61B 3/113, A61B 5/18

(54) **VALIDATION OF EYE TRACKING DATA**

(71) Applicant: DIVE Medical SL, 50018 Zaragoza (ES); Universidad De Zaragoza, 50009 Zaragoza (ES); Fundación Instituto de Investigación Sanitaria Aragón, 50009 Zaragoza (ES)
(72) Inventor: PUEYO ROYO, Mª Victoria, 50009 Zaragoza (ES); ORTÍN OBÓN, Marta, 50018 Zaragoza (ES); GUTIÉRREZ PÉREZ, Diego, 50009 Zaragoza (ES); MASIÁ CORCOY, Belén, 50009 Zaragoza (ES); ALEJANDRE ESCRICHE, Adrián, 50018 Zaragoza (ES); PAN, Xian, 50018 Zaragoza (ES); FANLO ZARAZAGA, Álvaro, 50018 Zaragoza (ES); VILELLA CENIS, Marina, 50018 Zaragoza (ES); PÉREZ ROCHE, Teresa, 50009 Zaragoza (ES); GIMENO VERDEJO, Mª Isabel, 50018 Zaragoza (ES)
(74) Representative: Smith, Richard

(57) **Abstract**

A computer-implemented method of validating eye tracking data for a system for performing eye tracking-based tests or tasks on a subject. The method comprises receiving (S8) eye tracking data of one or both eyes of a subject for each one of a sequence of visual stimuli (200), the eye tracking data indicative of one or more characteristics of the subject's gaze or position when each of the stimuli (200) were displayed. The method comprises receiving (S9) one or more subject indications comprising at least one of the following: the subject's age; one or more pathological or physiological conditions of the subject; and data representative of a calibration quality and/or attention level for one or more previous eye tracking-based tests or tasks performed by the subject. The method also comprises one or more of the following steps: a) quantifying a calibration quality of the eye tracking data and determining (S10) whether the calibration quality is lower than a predetermined minimum calibration quality associated with the subject indications, based at least on the eye tracking data the position of each visual stimuli and the subject indications; b) quantifying an attention level of the subject based on the eye tracking data, and determining (S10) whether the attention level of the subject is lower than a predetermined minimum attention level associated with the subject indications, based at least on the eye tracking data and the subject indications.

## Description

### TECHNICAL FIELD

The present disclosure relates to a computer-implemented method of validating eye tracking data for a system for performing eye tracking-based tests or tasks on a subject. The disclosure also relates to an apparatus and computer program for carrying out the method.

### BACKGROUND

Advancements in eye tracking technology have enabled eye tracking devices to provide a more accurate means of analysing a subject's ability to perform certain visual or cognitive tasks. Visual and cognitive tests and other tasks which were previously performed manually by a medical practitioner and depended on the subjective analysis of the medical practitioner can now be performed objectively by an eye tracking-based apparatus.

In order to be able to effectively analyse the eye tracking data, the subject must be focussed on the visual stimuli provided, the system must be correctly calibrated and the calibration must remain valid during the entire period where the eye tracking data is obtained. This may not always be the case and the medical practitioner may not notice that the subject has not been focussed on the task or has moved to a new position in which the calibration is no longer valid. In particular, in the case of non-collaborative and non-communicative subjects, it is even harder to ensure that the subject pays attention to the task, and the likelihood is higher that less attention will be paid to the task.

There is therefore a need to objectively and reliably obtain the best possible quality data for each subject when obtaining eye tracking data and to reliably determine when low quality data has been obtained.

### SUMMARY OF THE INVENTION

According to a first aspect of the invention, there is provided a computer-implemented method of validating eye tracking data for a system for performing eye tracking-based tests or tasks on a subject, comprising:
receiving eye tracking data of one or both eyes of a subject for each one of a sequence of visual stimuli, the eye tracking data indicative of one or more characteristics of the subject's gaze or position when each of the stimuli were displayed;
receiving one or more subject indications comprising at least one of the following: the subject's age; one or more pathological or physiological conditions of the subject; and data representative of a calibration quality and/or attention level for one or more previous eye tracking-based tests or tasks performed by the subject; and
one or more of the following:
   a) quantifying a calibration quality of the eye tracking data and determining whether the calibration quality is lower than a predetermined minimum calibration quality associated with the subject indications, based at least on the eye tracking data the position of each visual stimuli and the subject indications; and/or
   b) quantifying an attention level of the subject based on the eye tracking data, and determining whether the attention level of the subject is lower than a predetermined minimum attention level associated with the subject indications, based at least on the eye tracking data and the subject indications.

According to a second aspect of the invention, there is provided an apparatus configured to perform the method according to the first aspect, comprising:
- a display device configured to display the visual stimuli to the subject;
- an eye tracking device configured to track the gaze of the subject when the visual stimuli are displayed to the subject and to generate eye tracking data indicative of one or more characteristics of the subject's gaze or position; and
- one or more processors configured to control the display device to display the visual stimuli, to receive eye tracking data from the eye tracking device, and determine whether the eye tracking data is indicative of a lower quality than the predetermined minimum quality, based at least on the eye tracking data for each visual stimuli, and the one or more subject indications of the subject.

According to a third aspect of the invention, there is provided a computer program comprising computer-readable instructions which, when executed by a processor, cause the processor to execute a method according to the first aspect.

### BRIEF DESCRIPTION OF THE DRAWINGS

To enable better understanding of the present disclosure, and to show how the same may be carried into effect, reference will now be made, by way of example only, to the accompanying drawings, in which:
Fig. 1 shows a method for validating eye tracking data according to one or more embodiments.
Fig. 2A shows a schematic diagram of an apparatus according to one or more embodiments.
Fig. 2B shows a schematic front view of an apparatus according to one or more embodiments.
Figs. 3A to 3C show a sequence of a plurality of visual stimuli shown during a method according to one or more embodiments.

### DETAILED DESCRIPTION

As used herein, the term "eye tracking-based test" may be understood to mean any test which displays a sequence of visual stimuli and utilises qualitative or quantitative eye tracking data of a subject viewing the stimuli to determine the visual function or cognitive ability of the subject. The visual stimuli used for a given test may be configured as required in order to determine the particular aspect of visual function or cognitive ability of the subject (for example the colour, size, duration, shape etc. of each visual stimuli. or any other visual features of the stimuli or the background of the display may be varied depending on the type of test performed so that the gaze of the subject provides a marker for the performance of the subject in that test). Depending on the test performed, the visual stimuli may each be static (displayed at fixed position) or dynamic (moving over time, or the visual characteristics of the stimuli changing over time, for example pulsating, changing in colour, and any other change of the appearance of the visual stimulus).

As used herein, the term "eye tracking-based task" may be understood to mean any task which involves the display of a sequence of visual stimuli to be viewed by a subject, for which the subject's eyes are tracked in order to determine, measure, grade or otherwise analyse the performance of the task by the subject. The task may be part of a training session, therapy, a game, or any other task. As above, the visual stimuli used may vary depending on the type of task.

As used herein, the term "visual stimulus" may mean any physical or virtual object displayed to a subject. The visual stimuli may be displayed on any of the electronic display devices disclosed herein or may comprise physical objects displayed to the patient at predetermined positions set by a medical practitioner.

As used herein, the term "subject" may be understood to mean any person who is performing a task involving the appearance of visual stimuli. The subject may be a patient in a clinical setting performing one or more tasks relating to the calibration of an eye tracker in preparation for one or more tests of the subject's visual function or cognitive ability of the patient. Whilst the present disclosure is primarily directed towards determining whether the quality of eye tracking data obtained is acceptable in a clinical setting (for example during one or more tests of a patient's visual function or cognitive ability), the subject may be a user of a display device performing visual tasks in other circumstances.

As used herein, the term "static visual characteristic" may be understood to mean any characteristic of a visual stimulus which describes any aspect of the appearance of the visual stimuli at an instance in time. Static visual characteristics include, for example, the shape, size, colour or colours of the stimulus, the brightness or contrast of the stimulus.

As used herein, the term "dynamic visual characteristic" may be understood to mean any characteristics describing how the position or the static visual characteristics of the visual stimuli change over time. For example the stimulus may move around the screen or may pulsate (change in size over a time cycle), or any of the static visual characteristics may change over time (e.g. shape, size, colour(s), brightness, contrast).

Examples of eye tracking-based tests include:
- Visual acuity tests.
- Contrast sensitivity tests.
- Color perception tests.
- Oculomotor control tests.
- Visual field tests.
- Visual recognition tests.
- Strabismus detection and/or measurement tests.
- Pupil reaction tests.
- Object recognition tests.
- Preferential looking paradigm tests.
- Visual perceptual tests.
- Visual motor tests

As used herein, the term "eye tracking data" may be understood to mean any data which is indicative of one or more characteristics of the subject's gaze or position and from which one or more of the following can be determined: the precision or accuracy of the eye tracking device generating the data, and whether the subject is paying attention to an eye tracking-based task. The eye tracking data may comprise direct data indicating the one or more characteristics of the subject's gaze, or may comprise data from which the one or more characteristics may be derived or calculated. The eye tracking data may for example be gaze data which characterises the position or type of the gaze (for example fixation, saccade, pursuit, blinks, etc.) of one or both eyes of a subject. The eye tracking data may be qualitative or may be quantitative.

As used herein, the term "characteristic" in relation to the subject's gaze or position may be understood to mean any characteristic which describes a physical feature of the subject's gaze or position. Gaze characteristics may include the direction of the gaze in relation to any coordinate system, the location of the gaze relative to any coordinate system (e.g. relative to the eye tracker, the display or the stimulus), and the position of the eye(s) of the patient. Position characteristics may include the position or orientation of the patient's head, eyes, or pupils.

Examples of quantitative eye tracking data include:
- Position or direction of the gaze of the subject relative to the stimulus or relative to an absolute coordinate system.
- Position of the pupil(s) of the subject relative to the eye tracking device or relative to an imaginary tracking box projected towards the subject's face, or relative to another coordinate system.
- Size of the pupil(s).
- Image data of the eyes of the subject (the position of gaze can be determined from known image analysis algorithms of the face).
- Quantitative indicators of how well the eye tracking data fits to a calibration model of an eye tracker. For example, an eye tracker may comprise an automatic calibration algorithm which fits the eye tracking data of a subject obtained during a calibration process to an eye tracking model. How well the data is calibrated may be indicated by, for example, a normalised numerical value.
- The number or proportion of visual stimuli for which a calibration algorithm of an eye tracker fails (i.e. for which it is determined by the eye tracker that calibration was not possible).
- The number or proportion of visual stimuli for which the eye tracker needed to repeat due to a failure in calibrating the data for those stimuli (or the number of times the eye tracker issued a request to repeat a visual stimulus for a calibration point).
- How long each visual stimulus needed to be displayed during a calibration process before the calibration algorithm of the eye tracker successfully calibrated the calibration point.
- Vector formed by the pupil and the reflection of light in the eye.

Examples of qualitative eye tracking data include:
- For each data point, whether the subject is looking at the visual stimuli or not.
- For each data point, whether the subject is looking within a certain distance of the visual stimuli or not.
- For each data point, whether the subject's gaze falls on the display or not.
- For each data point, whether the pupil size is lower than a predetermined threshold value.

As used herein, the term "pathological or physiological condition" may be understood to mean any anatomical, mental or any other type of condition which affects the ability of the subject to perform an eye tracking-based task , or which affect the visual function of the subject (for example oculomotor control), or which affects any other aspect of the subject's vision or ability to concentrate on or view visual stimuli. The term may be referred to as "pathological condition" in the below, but the same meaning is intended.

As used herein, the term "non-communicative" in the context of a subject may mean any subject who is restricted in communicating effectively to the medical practitioner, either verbally or non-verbally. For example, the subject may be too young to communicate directly to the medical practitioner or may have a pathological condition which restricts the ability to communicate with the medical practitioner.

As used herein, the term "non-collaborative" in the context of a subject may mean any subject who is restricted in understanding or following the instructions of the medical practitioner. For example, the subject may be too young to understand instructions given by the medical practitioner or may have a pathological condition which restricts the ability to understand or follow the instructions of the medical practitioner.

As used herein, the term "calibration quality" in the context of eye tracking data may be understood to mean a measure of the reliability of the calibration of the eye tracking device used to generate the eye tracking data for assessing a subject's performance of an eye tracking-based test or task. The calibration quality may be affected by a poor initial calibration which results in a reduced precision and/or accuracy of the eye tracking device and therefore a reduced correlation between the location of the gaze measured by the eye tracking device and the actual position of the gaze. In such cases, the system may require recalibration. The calibration quality may also be affected by movement of the subject away from the original position of the subject when the calibration was performed. If the subject moves too far away from the original position, the calibration may be considered no longer valid as the errors caused by the changed position reduces the precision and/or accuracy of the eye tracking device. In such cases, the calibration quality may be improved without requiring recalibration. A poor calibration quality means that the subject's performance of a task cannot be reliably determined, whereas a high calibration quality means that the system is able to accurately determine the subject's performance of a task. Calibration quality can be quantified in numerous ways, some of which are described in further detail below.

As used herein, the term "attention level" may be understood to mean a measure of the attention paid by the subject to the display of the visual stimuli. It is preferable to measure the attention level of the subject because this helps to differentiate between poor performance of a task or test due to, for example, the subject's inability to see the visual stimuli due to a pathological condition, and poor performance due to the subject not paying attention to the visual stimuli. Attention level can be quantified in numerous ways, some of which are described in further detail below.

For the sake of brevity, the term "data reliability" may be used to refer to either calibration quality, attention level or a combination of the two.

It is noted that for all of the disclosed embodiments herein, the disclosure may apply to one or both of the subject's eyes. In the case where both of the subject's eyes are tracked, the data may comprise two datasets (one for each eye) or may comprise a single combined dataset of a combined gaze value (for example average value of the two eyes). Accordingly, when reference is made to a single eye, alternatively the same may be applied to both eyes. Conversely, when reference is made to both eyes or to "eyes" in plural, alternatively the same may be applied to only one eye. The skilled person will appreciate that any of the tests, tasks or calibrations disclosed herein may be applied to one or both eyes, and any disclosure otherwise should not be construed as limiting the scope of the invention.

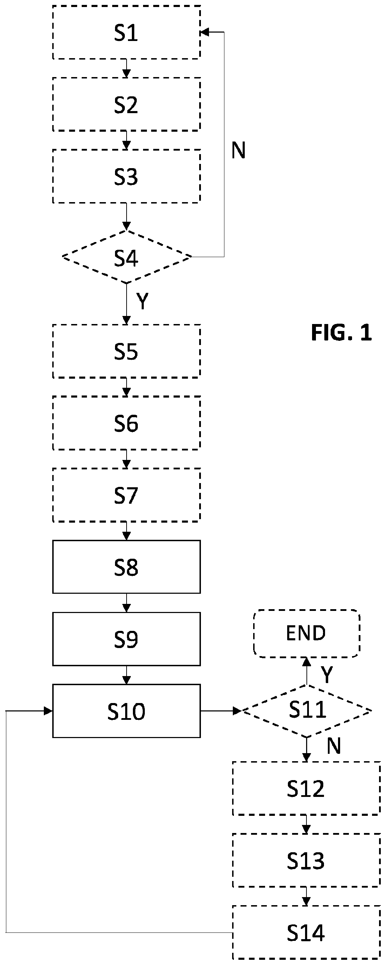

Fig. 1 shows a computer-implemented method according to one or more embodiments of the invention. In the illustrated method, boxes shown with dashed lines are optional. In some embodiments, the method begins (step S8) with receiving eye tracking data of one or both eyes of a subject for each one of a sequence of visual stimuli (i.e. a plurality of stimuli displayed one at a time in a sequence). The visual stimuli may be displayed at a plurality of spatial positions. The eye tracking data is indicative of one or more characteristics of the subject's gaze or position when each of the stimuli were displayed. Some or all of the plurality of spatial positions may be the same spatial position depending on the task to be performed. It is noted that the method does not require the eye tracking steps to be actually performed, for example the eye tracking data may be data previously generated by a separate device, which is received by the computer. In a subsequent step S9, the method comprises receiving one or more subject indications comprising at least one of the following: the subject's age; one or more pathological or physiological conditions of the subject; and data representative of the calibration quality and/or attention level for one or more previous eye tracking-based tests or tasks performed by the subject. The method then (step S10) determines whether the eye tracking data is indicative of a lower reliability than a predetermined minimum quality associated with the subject indications, based at least on the eye tracking data for each visual stimuli and the subject indications. Step S10 comprises one or both of: quantifying a calibration quality of the eye tracking data and determining whether the calibration quality is lower than a predetermined minimum calibration quality associated with the subject indications, based at least on the eye tracking data, the position of each visual stimuli and the subject indications; and/or quantifying an attention level of the subject based on the eye tracking data, and determining whether the attention level of the subject is lower than a predetermined minimum attention level associated with the subject indications, based at least on the eye tracking data and the subject indications. As previously discussed, both calibration quality and attention level affect the reliability of the eye tracking data in determining the subject's performance of the test or task. Optionally, the step S10 may also be based on the position of each visual stimuli if the characteristic of the eye tracking data is to be calculated in relation to the position of the stimuli. Advantageously, the method takes into account the subject indications to determine whether a higher reliability of the tracking data is possible, either by improving the calibration quality or by improving the attention level of the subject. For example, it has been observed that the average calibration quality and attention level of normative eye tracking data varies for subjects of different ages and/or pathological or physiological conditions. In particular, for example, subjects of a younger age tend to have a lower attention level and the highest expected attention level for the eye tracking data for a younger subject is generally lower than an older subject. In other examples, the subject may have a pathology which generally reduces the calibration quality. For each subject, in order to determine whether an improvement in calibration quality or in attention level for a test or task is possible, the data can be compared to reference data (by a rules-based algorithm or by a machine learning algorithm) given the subject indications for the subject provided. Therefore, the method is able to differentiate between subjects to indicate whether the calibration quality and/or attention level is lower than expected or not. For example, for identical eye tracking data of a given calibration quality and/or attention level, for some ages and/or pathological indications, it may be determined that the calibration quality and/or attention level is acceptable, whereas the same eye tracking data may be considered of a low calibration quality and/or attention level for other ages and/or pathological conditions. This is because the ability of the subject to hold their gaze and/or attention for the visual stimuli depends on these factors. It is especially important to automatically determine whether the data reliability is sufficiently high for non-communicative or non-collaborative subjects, because the practitioner is unable to effectively communicate instructions to the subject and the subject may be unable to correctly follow instructions for the visual tests. Verifying the data is therefore an important tool for the practitioner to determine that the best possible eye tracking data for each subject has been correctly obtained, and if not, a further test, task or calibration can be performed in order to improve the data reliability.

It is noted that steps S8 to S10 may be continuously performed in real time as new eye tracking data is generated. For example, the eye tracking data may be analysed for quality over a moving time window.

The age indication may be provided in years, months and/or days, and when the subject's age is provided as an indication, the reference data to which the eye tracking data is compared may be reference data based on subjects within the same age bracket as the subject. For example, the method may comprise determining which age bracket of a predetermined set of age brackets the subject falls into (a non-limiting example is 0-6 months, 6-12 months, 12-18 months, 18-24 months, 2-3 years, 3-4 years, 4-5 years, 5-6 years and over 6 years), and comparing the eye tracking data of the subject to reference data for subjects in the same age bracket, or using reference curves. The pathological or physiological condition may include any of those listed herein or any other relevant pathological or physiological condition and the data may be compared to reference data for subjects having the same pathological or physiological condition. When both age and pathological/physiological conditions are provided, the eye tracking data may be compared to reference data from subjects within the same age bracket and having the same pathological/physiological condition(s). Alternatively or additionally, when the data representative of the data quality for one or more previous eye tracking-based tests or tasks performed by the subject is provided, this may be any previous eye tracking data generated prior to the received eye tracking data which is representative of a calibration quality and/or attention level for one or more previous eye tracking-based tests or tasks, and in some examples may comprise one or more statistical parameters characterising said previous eye tracking data, or a quantitative quality parameter derived from said previous eye tracking data (for example a secondary parameter as disclosed herein), representative of a calibration quality and/or attention level for one or more previous eye tracking-based tests or tasks. When the age and/or pathological and/or physiological condition is provided, advantageously, the method can determine whether the eye tracking data is of an acceptable quality for the subject even if no previous eye tracking-based data has been obtained for the subject unlike in the embodiments where only previous test or task performance of the subject is used, which requires a previous eye tracking test or task to have been performed on the same subject.

It is noted that the determining step S10 could be performed by a rules-based algorithm or by a machine learning algorithm. In embodiments where a rules-based algorithm is used, the step may comprise a first step of receiving or calculating, for one or both eyes, one or more statistical parameters describing a plurality of data points of the eye tracking data, or one or more secondary parameters derived from said one or more statistical parameters. The statistical parameters may include, for example one or more of: the mean, median, mode, standard variation, or variance of any of the quantitative eye tracking data for each stimulus when quantitative eye tracking data is generated; parameters describing a best fit of the quantitative data (for example describing the normal distribution of data points); the bivariate contour ellipse area (BCEA), known in the art, and, in the case of the qualitative data, the proportion of data points within one or more of the qualitative data categories (for example the proportion of data points where the subject was looking at the stimulus or looking at the screen). The data may also comprise one or more sets of data with associated categories for the type of gaze characterising the sets of data (for example saccades, fixations, smooth pursuit movements), such that the sequence of eye tracking data is characterised by the type of gaze over time.

The one or more secondary parameters may be one or more parameters generated according to a predetermined equation which is a function of one or more of the statistical parameters (for example a single secondary parameter which is a function of all of the statistical parameters such that the statistical parameters are characterised by a single numerical value). In a second step, a comparison is performed for the one or more statistical parameters or secondary parameters to one or more predetermined threshold values associated with the subject indications, the one or more threshold values indicative of acceptable calibration quality and/or attention level for the given subject indications. For example, each of the one or more statistical parameters and/or secondary parameters is compared to a threshold value for that parameter which is the threshold defining the boundary between indicating an acceptable or unacceptable calibration quality and/or attention level for the subject indications. For example, for any given age and/or pathological and/or physiological conditions, reference data may be obtained for subjects in the same age bracket and/or having the same pathological and/or physiological conditions, and the average statistical/secondary parameters may be determined from the reference data. A predetermined threshold can then be defined based on the reference statistical/secondary parameters (for example, one standard deviation away from the average reference value, or a percentage difference from the average reference value), beyond which the parameter is considered of a low calibration quality and/or attention level for the age and/or pathological or physiological conditions. It is noted that depending on the statistical/secondary parameter, a higher numerical value may indicate a higher or a lower calibration quality or attention level, and for a given statistical parameter or secondary parameter this will be apparent to the skilled person. Therefore, a value above or below the threshold may be indicative or acceptable or unacceptable data reliability, or vice versa. If the subject indication comprises data representative of the calibration quality or attention level for one or more previous eye tracking-based tests or tasks performed by the subject, then the same statistical or secondary parameters may be calculated for the previous data and a calibration quality and/or attention level threshold may be set accordingly (again, for example one standard deviation from the statistical parameter, or a percentage difference such that a substantial deviation from a previous level of calibration quality or attention level indicates that more reliable data could be obtained). In some embodiments where data representative of the data quality of a plurality of previous eye tracking-based tests performed by a subject over time is used, the calibration quality or attention level threshold may be set by analysing a trend in the data over time. For example, if the data indicates that over time the reliability of the data has improved (for example because the subject is receiving treatment or therapy for a pathology), then a higher calibration quality and/or attention level threshold may be set for the eye tracking data taking into account this trend. Conversely, if the reliability of the previous data decreases over time (for example due to a degenerative disease), then a lower calibration quality and/or attention level threshold may be set for the eye tracking data taking into account this trend. In a third step, if a predetermined number of the one or more statistical parameters or secondary parameters do not meet the one or more associated predetermined threshold values, the method comprises determining that the eye tracking data is of a lower reliability than a predetermined minimum reliability (calibration quality and/or attention level), and otherwise determining that the eye tracking data is an acceptable reliability. The predetermined number may be at least one of the statistical parameters and/or secondary parameters that are calculated (i.e. one or more of the calculated parameters do not meet the predetermined threshold), or the predetermined number may be two or more of the parameters, or three or more, and so on, or all of the calculated parameters (i.e. all of the calculated parameters do not meet the predetermined threshold). In some embodiments, the method comprises comparing the data to at least one calibration quality threshold and at least one attention level threshold.

In an alternative embodiment, step S10 is performed by an Artificial Intelligence (AI) algorithm such as a machine learning algorithm. In such embodiments, the step S10 comprises inputting the eye tracking data or statistical or secondary parameters of the eye tracking data, optionally the position of the visual stimuli, and the subject indications into an AI algorithm, wherein the AI algorithm is trained to determine whether the eye tracking data is of an acceptable or a lower reliability (calibration quality and/or attention level) than a predetermined minimum quality associated with the subject indications compared to reference subject data having the same subject indications. The AI algorithm can be any suitable algorithm as known in the art, and may include any supervised, unsupervised, semi-supervised and reinforced learning algorithms. When the subject indications comprise the subject's age and/or one or more pathological/physiological conditions, the training data for the AI algorithm comprises a plurality of sets of data for subjects of each age bracket or pathological/physiological condition (or each combination thereof, as described above). Each data set may comprise a plurality of data points indicating the location of the gaze relative to a stimulus, and/or one or more statistical parameters describing the plurality of data points, and/or one or more secondary parameters derived from said one or more statistical parameters, and/or qualitative data for a plurality of data points as previously described, and/or statistical parameters describing the qualitative data as previously described. The training data further comprises, for each data set, an indication of the reliability (calibration quality and/or attention level) of the data set for the subject's age and/or pathological and/or physiological condition. The indication may be, for example, a binary value, a quantitative number in continuous or discrete form, one of a plurality of labels (for example "low", "medium", "high"), or any other suitable indication. The machine learning algorithm can then be trained to determine whether for new input data, given the subject indications (e.g. age, pathological/physiological condition), or combination thereof and the eye tracking data and/or statistical parameters and/or secondary parameters of the eye tracking data, whether the reliability of the eye tracking data is above or below the minimum quality given the age and/or pathological and/or physiological condition.

When the subject indications comprise data representative of the data reliability (calibration quality and/or attention level) for one or more previous eye tracking-based tests or tasks performed by the subject, the AI may be trained using training data comprising, for a plurality of subjects: the eye tracking data or statistical or secondary parameters of the eye tracking data, data representative of the data reliability for one or more previous eye tracking-based tests or tasks performed by the subject; and an indication of whether the eye tracking data or statistical or secondary parameters of the eye tracking data are of an acceptable reliability compared to the data reliability of the one or more previous eye tracking-based tests or tasks performed by the subject. The indication may be, for example, a binary value, a quantitative number in continuous or discrete form, one of a plurality of labels (for example "low", "medium", "high"), or any other suitable indication.

In some embodiments the method terminates after the determining step S10 is executed regardless of the outcome of step S 10. In some embodiments, a notification is communicated to the user regarding the outcome of step S 10 (for example an audio or visual notification that the calibration quality and/or the attention level is higher or lower than the predetermined minimum calibration quality and/or attention level). Optionally, in some embodiments, the method may further comprise, if it is determined that the eye tracking data is of a higher calibration quality and/or attention than a predetermined minimum calibration quality and/or attention level (S11), terminating, and if it is determined that the eye tracking data is of a lower calibration quality and/or attention level than a predetermined minimum calibration quality and/or attention level (S11), obtaining new eye tracking data by displaying (S12) to the subject a second plurality of visual stimuli, the visual stimuli displayed individually in sequence, the visual stimuli having a plurality of spatial positions. Some or all of the plurality of spatial positions may be the same spatial position depending on the task to be performed. The new eye tracking data may be obtained in order to recalibrate the eye tracking device when it is determined that the calibration quality is unacceptable (or to obtain data of a higher calibration quality without recalibrating the eye tracking device, for example by repositioning the subject). The new eye tracking data may be obtained in order to obtain improved eye tracking data for a given test or task when it is determined that the attention level is unacceptable. The method may further comprise tracking (S13), by an eye tracking device, the gaze of one or both of the eyes of the subject for each of the visual stimuli. The eye tracking device may be any eye tracking device capable of tracking the position of one or both of the subject's eyes and placed in a known position relative to the visual stimuli (for example any commercially available eye trackers, or any computing device comprising an imaging device such as a camera and suitable software for tracking the eye position based on the captured image), or of generating any other eye tracking data indicative of one or more characteristics of the subject's gaze or position. The method may further comprise generating (S14), based on the tracked gaze by the eye tracking device, the eye tracking data indicative of one or more characteristics of the subject's gaze or position. In some embodiments, step S10 may be performed for the new eye-tracking data. Steps S 1 1 to S14 may be repeated a plurality of times (e.g. twice or three times or until an acceptable reliability of data has been obtained). It is noted that steps S11 to S14 may be performed with or without preceding steps S1 to S7 as described in further detail below. In embodiments where steps S11 to S14 are performed without preceding steps S5 to S7, the received data in step S8 may include an indication of the visual characteristics of the stimuli shown to the subject, so that similar visual stimuli can be shown in steps S12 to S14.

In some embodiments where steps S12 to S14 are performed, the second plurality of visual stimuli have different static or dynamic visual characteristics used to generate the eye tracking data which, in step S10, has been determined to have a calibration quality and/or attention level lower than the predetermined minimum calibration quality and/or attention level. Alternatively or additionally, if the plurality of visual stimuli each have different static or dynamic visual characteristics between themselves, the plurality of stimuli may have a smaller overall range of different static or dynamic visual characteristics compared to the visual stimuli used to generate the eye tracking data of an unacceptable calibration quality and/or attention level from step S10. Alternatively or additionally, the second plurality of visual stimuli are displayed over a smaller field of view than for the visual stimuli used to generate the eye tracking data of an unacceptable calibration quality and/or attention level in step S10. Alternatively or additionally, one or more audio stimuli are provided to the subject during the sequence of the second plurality of visual stimuli. Advantageously, this allows the visual stimuli to be adapted to the subject in order to more effectively attract the attention of the subject. For example, it may be that the subject did not view the visual stimuli because the stimuli were too small for the subject to notice, or because the subject has a pathological condition which causes the stimuli to be less noticeable (for example the subject is colour blind). In some cases where the subject is unable to understand that a test or task is being performed (for example if they are very young), they may only sporadically pay attention to the visual stimuli, meaning that the attention level of data is low. Accordingly, changing the visual characteristics or providing other stimuli in combination with the visual stimuli may attract the attention of the subject in order to obtain the required eye tracking data of an acceptable attention level. In some embodiments where steps S12 to S14 are repeated several times as described above, the visual characteristics may change each time steps S12 to S14 are repeated. Especially in the case of non-communicative or non-collaborative subjects, repetition of the test or task with different visual characteristics increases the likelihood that eye tracking data of a sufficient quality is obtained. Specific examples of the changes for the second plurality of stimuli include:
- showing stimuli of larger sizes;
- showing stimuli which move at lower speeds;
- displaying the second plurality of stimuli over a smaller field of view, i.e. making the stimuli appear not as far from each other as before (increasing the likelihood that the subject is able to notice a second stimulus when their gaze is in the position of the previous stimulus);
- emitting prompting audios or music to attract the subject's attention;
- showing the stimuli with additional dynamic visual characteristics such as vibrations, pulsation or other movements;
- showing the stimuli wherein the stimuli change colour more often than in the previous plurality of stimuli.
- increasing the brightness and/or contrast of the stimuli.
- reducing the range of visual characteristics between the stimuli, for example restricting all of the stimuli to a range of visual characteristics for which good quality data was obtained for the subject., or reducing the range of visual characteristics to a range which is generally easier to see for subjects.
- reducing the time duration of the test(s) or task(s).

It is noted that in any of the embodiments disclosed herein, the eye tracking data may be obtained during calibration of an eye tracking device. In such embodiments the plurality of visual stimuli may be displayed in order to calibrate the position of the subject's eyes with the measurements made by the eye tracking device, and the determining step determines whether the calibration quality of the calibration is of a lower quality than the predetermined minimum calibration quality for any eye tracking-based task or tests to be performed subsequently, given the subject indications. In some embodiments, the eye tracking data may be obtained during an eye tracking-based test or task, cognitive test or task or visual therapy performed on the subject. In such embodiments, the determining step determines whether the calibration quality and/or attention level of the eye tracking data is of a lower calibration quality and/or attention level than the predetermined minimum calibration quality and/or attention level for the visual test or task, cognitive test or task or visual therapy for the subject indications.

In some embodiments, the method may comprise the steps of actually generating the eye tracking data. In particular, the method may comprise the steps of displaying (S5) to a subject, a plurality of visual stimuli, the visual stimuli displayed individually in sequence, the visual stimuli optionally having a plurality of spatial positions. As previously discussed, the plurality of visual stimuli may be physical objects placed sequentially in predetermined positions relative to the subject by the practitioner, or may be displayed on an electronic display device such as those described herein. One or more audio stimuli may be provided in addition to the visual stimuli (for example when each of the stimuli are displayed, or according to the subject's gaze as described in further detail below). The method may further comprise tracking (S6), by an eye tracking device, the gaze of one or both of the eyes of the subject for each of the visual stimuli. The eye tracking device may be any suitable device for eye tracking as disclosed herein. The method may further comprise generating (S7), based on the tracked gaze by the eye tracking device, the eye tracking data characterising the position of the gaze of one or both eyes relative to the displayed stimulus during the display of each visual stimuli.

In some embodiments, the generated eye tracking data may exclude data obtained within a predetermined amount of time after the initial appearance of each of the stimuli. For example, eye tracking data obtained within 1 second after the initial appearance of each stimuli may be excluded. Alternatively or additionally, in some embodiments the generated eye tracking data may exclude eye tracking data obtained within a predetermined amount of time before the disappearance of each of the stimuli. For example, eye tracking data obtained within 1 second before the disappearance of the stimuli may be excluded. This reduces the likelihood that eye tracking data wherein the subject has not yet noticed or tracked the new visual stimuli, or has lost attention, artificially lowers the reliability of the eye tracking data.

In some embodiments, once the system is calibrated, the apparatus may be configured to detect whether the subject is viewing the visual stimuli or not, or whether the subject saw each of the visual stimuli at any time. This may be calculated based on a preset algorithm performed on the eye tracking data as known in the art. In some embodiments, if it is determined by the eye tracking device that the subject has not viewed the visual stimuli for a predetermined amount of time, it may be determined that the subject is not viewing or has not seen the visual stimuli.

Alternatively or additionally to determining whether the subject is viewing the visual stimuli or not, some eye tracking devices may provide a real-time indication about whether valid gaze data has been obtained. The real-time indication may be continuously monitored. In some embodiments, if there has been no valid eye tracking data obtained by the eye tracking device for a predetermined amount of time, it may be determined that the subject is not paying attention. Alternatively, the eye tracking data may comprise any other data which is indicative of whether the patient is paying attention as disclosed herein, which may be continuously monitored in real time to determine whether the subject is paying attention.

In embodiments where the system comprises an algorithm to detect whether the subject is viewing or was able to see the visual stimulus or not, one or more display characteristics (static and/or dynamic) of the visual stimulus may change when it is determined that the subject is looking at the visual stimulus, in order to maintain the attention of the subject. The one or more display characteristics may be changed in real time (i.e. whilst the subject is viewing the stimulus) or may be changed after the stimulus has disappeared or after the complete task has been performed (i.e. to provide positive feedback to the subject at the end of the task if the task was successfully performed or at the end of the stimulus being displayed). Alternatively or additionally, audio stimuli may be provided when it is determined that the subject is looking at or was able to see the visual stimulus, in order to maintain the attention of the subject. Alternatively or additionally, one or more display characteristics (static and/or dynamic) of the visual stimulus may change when it is determined that the subject is looking away from the visual stimulus, in order to maintain the attention of the subject. Alternatively or additionally, audio stimuli may be provided when it is determined that the subject is looking away from the visual stimulus, in order to maintain the attention of the subject. Specific examples include:
- displaying a static or moving (e.g. rotating) star or other encouraging symbol about the visual stimulus and/or emitting a sound when the subject is determined to be looking at the visual stimulus, or after having looked at the visual stimulus; and/or
- increasing the brightness of the visual stimulus when the subject is looking at the stimulus; and/or
- emitting a sound when the subject begins to look at the stimulus and/or when the subject begins to look away from the stimulus; and/or
- incrementally increasing the size of the visual stimulus when the subject is looking away from the visual stimulus, optionally returning the visual stimulus to its original size when the subject is looking at the visual stimulus (in order to maintain the difficulty of the test or task); and/or
- where the visual stimulus is configured to move during the test or task, maintaining the visual stimulus in a static position until the subject is determined to look at the stimulus, and then beginning movement of the visual stimulus once the subject is looking at the stimulus.

In some embodiments where the calibration quality and/or attention level is continuously monitored over a moving time window, when it is determined that the eye tracking data is indicative of a lower calibration quality and/or attention level than the predetermined minimum calibration quality and/or attention level, the task or test is paused. Optionally, the task or test is resumed when it is determined that the eye tracking data has returned to an acceptable calibration quality and/or attention level for the subject. When the process of generating the eye tracking data is resumed when the calibration quality and/or attention level returns to an acceptable level, the process may be resumed by, for example:
- displaying the same stimulus which was being displayed when it was determined that the subject's gaze was away from the visual stimulus; or
- discarding the data for the stimulus which was being displayed when it was determined that the subject's gaze was away from the visual stimulus and displaying an additional visual stimulus at the end of the sequence of visual stimuli;
- resetting the process to a predetermined amount of time or a predetermined number of visual stimuli before the process was paused; or
- continuing the sequence of stimuli from the point where the process was paused and optionally discarding eye tracking data obtained while the process was paused, optionally further discarding eye tracking data obtained a predetermined amount of time before the process was paused and after the process was resumed.

In some embodiments, prior to steps S5 to S7, the method may comprise tracking (S1), via the eye tracking device or a device for tracking the subject, the position of the subject. The method may further comprise indicating (S2) the position of the subject on a display device. The position of the subject on the display device may be indicated by displaying an image of the subject or a drawing or virtual representation of the subject (such as a rendered animated face) representing the position of the subject in front of the display device. The position of the subject may be calculated by the eye tracking device or by a computing device connected to the eye tracking device, using the eye tracking data generated by the eye tracking device and the known position and orientation of the display device and the eye tracking device relative to one another, and thus calculating the position of the subject in front of the display device (i.e. the position of the reflection of the subject if the display device were a mirror). Such calculations are known in the art. The method may further comprise overlaying (S3) a subject positioning guide on the display device to indicate the correct position of the subject for obtaining the eye tracking data. The positioning guide may be positioned on the display device so that once the image of the subject or the representation of the subject aligns with the guide, the subject is in the correct position. The guide may be any suitable form or shape, for example a border shaped as the outline of a face or two circles configured to align with the eyes of the subject or representation. The guide may be positioned until (S4) it is determined that the subject is correctly positioned, at which point the method may proceed to step S5.

The systems and methods disclosed herein are well-suited to obtain the highest expected calibration quality and/or attention level of eye tracking data for a given set of subject indications. It is noted that in some cases, the subject may be held in place by a guardian or care such that the eye tracker may detect a plurality of pairs of eyes within its field of view. For example, the subject may be an infant held in the lap of an adult. In such cases, the method may comprise indicating that a plurality of eyes have been detected. Alternatively or additionally the method may comprise generating the eye tracking data for the lowermost pair of eyes which are the subject's eyes. This reduces the amount of superfluous data generated and reduces errors caused by tracking the incorrect pair of eyes.

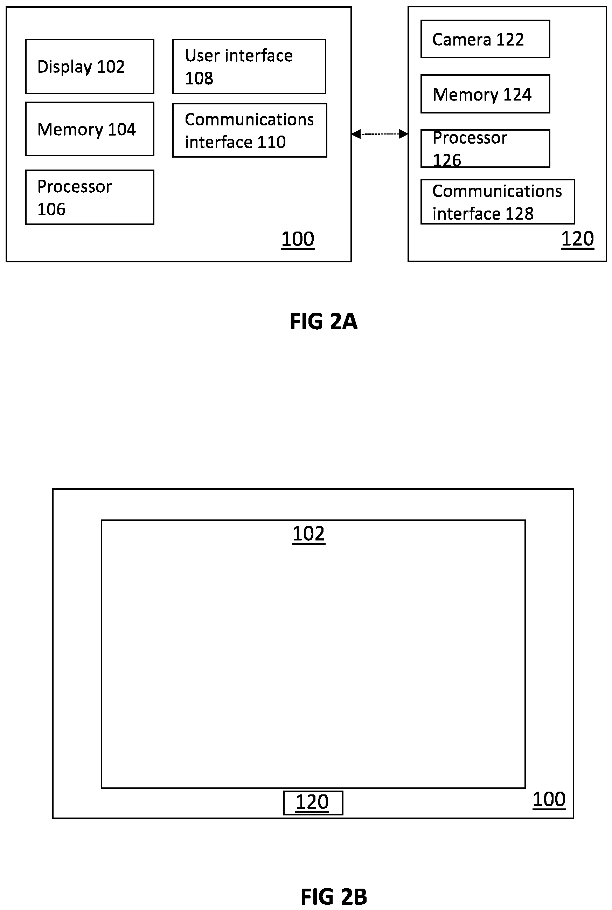

Fig. 2A shows an apparatus for carrying out the methodology of the invention according to one or more embodiments. The apparatus comprises an eye tracking device 120. The eye tracking device may be any suitable device as disclosed herein. The eye tracking device comprises an imaging device, for example a camera, 122, a memory 124, processor 126 and communications interface 128. The imaging device 122 may be any suitable imaging device for imaging the eyes of the subject such that they may be tracked. The memory 124 may comprise instructions for detecting and calculating the position of the subject's eyes relative to the displayed visual stimuli, or relative to any other coordinate system using the image data generated by the imaging device 122. The memory may comprise a sequential list of predetermined positions for the visual stimuli relative to the eye tracking device (for example the positions on the display device or the positions of the objects to be displayed by the practitioner), or the device may receive indications of these positions manually via a user interface (not shown). The processor 126 may be configured to calculate the position of the subject's eyes as a function of time, from the image data collected by the imaging device and the positions of the visual stimuli. The eye tracking device 120 may further comprise a communications interface 128 for communicating the imaging data generated by the imaging device 122 or any of the data calculated by processor 126. The eye tracking device 120 may be configured to perform steps S1, S6, S7, S13 and S14.

It is noted that whilst the memory 124 and processor 126 are provided as part of the eye tracking device, these components may be provided remotely to each other and to the imaging device 122, with the data being transmitted between the components by any suitable wired or wireless connections (e.g. USB, internet or Bluetooth).

The apparatus may further comprise an electronic display device 100 for displaying the plurality of visual stimuli. The display device 100 may comprise any suitable electronic display 102 as known in the art. The display device 100 may comprise a memory 104 comprising instructions for displaying a plurality of visual stimuli on the display 102, as well as controlling the visual characteristics of the visual stimuli and providing any other stimuli (for example in embodiments where the display device 100 comprises or is connected to a speaker and auditory stimuli are provided in conjunction with the visual stimuli). The display device 100 may further comprise a processor 106 for processing or calculating any of the data such as the position of the subject's eyes based on the image data generated by imaging device 122, or for rendering the guide and the image or representation of the subject and determining when the subject is aligned with the guide in steps S2 to S4. The display device 100 may further comprise a user interface 108 (for example a GUI) for controlling the device, such as selectively initialising the calibration or eye tracking-based test or task. The user interface 108 may further be used to select in which way to change the test, task or calibration, for example the visual characteristics or other stimuli to be provided in step S12. The user interface 108 may further be used to input the subject indications or the memory 104 may comprise the subject indication. The display device 102 may comprise a communications interface 110 to communicate with external devices, for example to communicate to an external device the results of the calibration or eye tracking-based test or task, or to indicate that the calibration quality and/or attention level is lower than the predetermined calibration quality and/or attention level, or to receive imaging or other data from the eye tracking device 120 or any other data from eye tracking device 120 such as data points regarding the subject's gaze. It is noted that in some embodiments, the display device 100 may have an eye tracking device integrated, as shown in Fig. 2B. In such cases the memory 124 and processor 126 may be memory 104 and processor 106 and the communications interface 128 may be interface 110. The display device may be used to display any of the visual stimuli described in the methods disclosed herein, or to calculate whether the calibration quality and/or attention level is above the predetermined minimum calibration quality and/or attention level.

It is noted that in embodiments where display device 100 is provided, the functionality of the memory 124 and processor 126 may instead be carried out by memory 104 and processor 106 and the eye tracking device 120 may not comprise memory 124 or processor 126.

The memories disclosed herein may comprise any suitable storage medium such as an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

The processors disclosed herein may comprise any suitable processor including a CPU, GPU, microprocessor, distributed processing apparatus or any other suitable processing apparatus.

It is noted that the above-described embodiments of the eye tracking device 120 and the display device 100 are exemplary only and methods of tracking a user's eyes using an eye tracking device are known in the art. Any such eye tracking devices and/or display devices may be used in place of the specific embodiments disclosed herein. For example, any and all of the components of display device 100 and eye tracking device 120 may be provided in a single device.

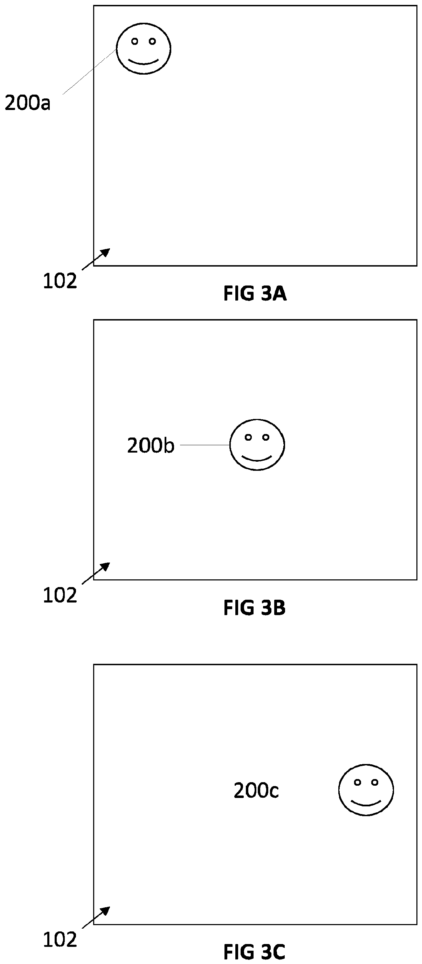

Figs. 3A to 3C show an exemplary sequence of visual stimuli that may be displayed on display 102 to obtain the eye tracking data. A first visual stimulus 200a may be displayed at a first location on the display 102. Subsequently, a second visual stimulus 200b may be displayed at a second location on the display 102. Subsequently, a third visual stimulus 200c may be displayed at a third location on the display 102. It will be understood that the visual stimuli 200 may be the same or different in shape, colour, size, duration in which they are displayed and so on. The example shown in Figs. 3A to 3C is illustrative only and it will be appreciated that the sequence of visual stimuli may be any suitable sequence suitable for obtaining eye tracking data of the subject. The sequence of visual stimuli may be for an eye tracking-based test or task, for example to test a certain aspect of visual function of the subject or to test the cognitive abilities of the subject or to provide visual therapy to the subject. As the location of each stimulus is known, and the positioning of the eye tracking device relative to the display 102 is known, the subject's gaze relative to each stimulus when they are displayed can be readily calculated, as known in the art, once the eye tracking device 120 is calibrated.

In some embodiments, there is provided a computer program comprising computer-readable instructions which, when executed by a processor, cause the processor to execute any of the methods disclosed herein. The computer program may be comprised in the memory of any of the devices disclosed herein or may be comprised in an external memory communicatively connected to any of the devices disclosed herein. The computer program may be executed on hardware and/or software.

It is noted that in any of the embodiments disclosed herein, the sequence of visual stimuli displayed to the subject (for example in steps S5 or S10) may be adapted based on the received subject indications. For example, the size or any other characteristic of the visual stimuli may adapted based on the age of the subject, or any pathological condition or physiological condition of the subject, Similarly, any other aspect of the displayed visual stimuli (sequence, time duration of each stimuli displayed, sounds associated with each visual stimulus, any static or dynamic visual characteristics) may be adapted based on the received subject indications. The different characteristics for the visual stimuli and any associated sounds for each combination of patient indications may be stored as computer-readable instructions in the memory of any of the devices disclosed herein.

### Examples of the use of eye tracking data

Different types of eye tracking data are now discussed in relation to determining whether the eye tracking data is indicative of a lower quality than a predetermined minimum quality according to the invention.

In some embodiments, the eye tracking data may comprise a plurality of data points representative of the position or direction of the gaze of the patient relative to each stimulus or relative to an absolute coordinate system. In such embodiments, the data points corresponding to being within a range of the displayed stimulus or on the display may be related to good attention level, because it indicates at least that the subject was paying attention to the display when the stimuli were displayed. The attention level can be quantified by, for example, calculating the number or proportion of data points for which the subject's gaze falls within an area considered to correspond to the subject paying attention (for example the gaze falling on the display). In other examples, it may be assumed that the subject is paying attention when the data is available, because the eye tracking data may be recorded when the subject is looking toward the display and not recorded when the subject is looking away. This data can be compared to the expected data for the subject indications (for example the maximum expected attention level may vary depending on the subject indications, and so it can be determined whether a better attention level could be obtained for the subject). In some embodiments, the location of the gaze can be compared to the location of the stimulus to determine whether the calibration of the eye tracker is correct. For example, the location of the gaze may be spread over a small angular range, meaning that the subject is focussed on a point on the display (and therefore that the stimulus has been observed), but that the location is, on average, a large distance away from the location of the stimulus. There may also be a consistent difference between the location of the gaze and the location of each stimulus. This could indicate that the calibration of the data is of a low calibration quality. The calibration quality may be quantified based on, for example, a normalised function dependent on the difference between the location of the gaze and the location of the stimuli, or any other suitable quantification method of calibration quality as known in the art. The calibration quality may depend on the pathology of the patient. For example, for patients with strabismus, the location of the gaze of one eye may be a large distance away from the stimulus, whereas in patients without strabismus this would indicate that the data is of a low calibration quality. Therefore, for any given data, the assessment of whether the data reliability is high or low may depend on the subject indications.

In some embodiments, the eye tracking data may comprise a plurality of data points representative of the position of the pupils of the subject relative to the eye tracking device, relative to an imaginary eye tracking box projected towards the subject's face, or relative to any other coordinate system such as the position of the subject's eyes when a calibration of the eye tracking device was performed. This data is indicative of good calibration quality when the pupils are located within a predetermined area of the coordinate system associated with the working range of the eye tracking device. The predetermined area may be defined by an area surrounding the location of the subject's eyes when the eye tracking device was calibrated. If the location of the eyes deviates significantly from this location, the accuracy of the calibration decreases. The predetermined area may be selected based on the minimum acceptable calibration accuracy. When the eyes are within the predetermined area, the eye tracking device is able to obtain accurate eye tracking data based on the calibration whereas outside of the predetermined area the eye tracking device does not track the eyes accurately and the calibration may be invalid, decreasing the accuracy of the data. The calibration quality of the data may be quantified, for example, by taking the number or proportion of data points within the predetermined area, or any other function of the data points such as a parametric calculation of the average position of the pupils. Furthermore, when the pupils are located within a predetermined area, it may be indicative that the subject is attentive to the stimuli as they are looking towards the display. Therefore, the level of attention of the subject can be determined from this data (and can be quantified, for example, by taking the number or proportion of data points for which the position of the pupils were inside the predetermined area). It is noted that the predetermined area for which the calibration is considered valid may be different in size for the predetermined area for which the attention level is considered valid.

In some embodiments, the eye tracking data may comprise data indicative of the size of the subject's pupils in absolute terms or relative to a reference size of the pupils (for example the size of the pupils when the eye tracking device was calibrated for the subject). The data may comprise, for each data point, an indication of whether the pupil size is smaller or larger than predetermined threshold values. The size of the subject's pupils is an indicator of how close or far from the display and/or eye tracking device the subject is. This data is indicative of good calibration quality when the size of the pupils is within a predetermined range (for example within a predetermined range relative to the size of the pupils when the calibration was performed), such that it can be inferred that the position of the subject has not changed substantially between when the eye tracking device was calibrated and when the eye tracking data was obtained. The calibration quality can be quantified based on this data by, for example, calculating the number or proportion of data points for which the size of the pupils were within the predetermined range for which the calibration is valid. Similarly, the size of the pupils being within a predetermined range may indicate that the subject is attentive to the stimuli as they are looking towards the display at the right distance from the display, and the attention level may be quantified in the same manner. The predetermined ranges for the calibration quality and the attention level may be the same or different.

In some embodiments, the eye tracking data may comprise image data of the eyes of the subject. An image analysis algorithm may analyse the image data to determine a plurality of data points for one or more of: the position of the face, the position of the pupils, the position of the gaze or the direction of the gaze relative to any coordinate system. The position or the size of the pupils, the position of the gaze and the direction of the gaze can be assessed for calibration quality and/or attention level as disclosed herein. Similarly, the position of the face may be compared to a predetermined area for which the calibration quality is assumed to be acceptable and/or for which the attention level is acceptable, and the calibration quality and/or attention level may be similarly quantified.

In some embodiments, the eye tracking data may comprise one or more quantitative indicators of how well the eye tracking data fits into a calibration model of the eye tracking device. This data is a direct indicator about how well the system is calibrated for the subject, and may also be an indicator of the attention level of the subject during calibration. The best expected calibration quality for a given subject may vary depending on the subject (for example the attention of the subject may vary, or the quality of calibration may vary depending on the subject), so it can be determined whether a better quality calibration or attention level is achievable based on previous data for subjects having the same or similar subject indications.

In some embodiments, the eye tracking data may comprise data indicative of one or more of: the number or proportion of visual stimuli displayed during a calibration process for which a calibration algorithm of an eye tracker fails; the number or proportion of visual stimuli for which the eye tracking needs to be repeated due to a failure in the calibration algorithm of the eye tracking device for those stimuli; how long each visual stimulus needed to be displayed during a calibration process before the calibration algorithm of the eye tracking device successfully calibrated the calibration point. The calibration algorithm may fail for a number of reasons, including a lack of attention paid by the subject, or a pathology of the subject which causes the algorithm to fail more often. The number of calibration failures may therefore depend on each patient such that re-calibration may or may not be expected to provide improved calibration quality and/or attention level. The number of repetitions and how long each stimulus was displayed during calibration are equally indicative of the calibration quality (more repetitions and/or a longer display time increase the probability of a low quality calibration).

In some embodiments, the eye tracking data may comprise data indicative of a vector formed by the pupil and reflection of light in the eye. The existence of this vector and its positioning within predetermined acceptable ranges and with changes within predetermined acceptable ranges is related to the attention of the subject and to good eye tracking quality. Further, the location of the gaze can be derived from this data from which it can be determined whether the subject was paying attention and whether the calibration quality is good, as previously disclosed.

In some embodiments, the eye tracking data may comprise data indicative of whether for each data point, the patient is looking at the visual stimuli or not, or within a predetermined distance of the visual stimuli or not. For stimuli where the patient is expected to see said stimuli, an indication that the patient is looking at the stimulus is a clear indicator that the eye tracking calibration quality is good and that the subject is paying attention to the stimuli. The data points may be quantified by determining the number or proportion of data points where the subject was looking at the stimuli, from which it can be determined whether the calibration quality and/or attention level is as good as the predetermined acceptable calibration quality level and/or attention level given the subject indications.

In some embodiments, the eye tracking data may comprise data indicative of whether for each data point, the patient's gaze falls on the display or not. This is related to whether the subject was paying attention to the display and therefore likely to notice the stimuli if they were visible to the subject. Accordingly, this data is a good indicator of whether the subject was paying attention when the stimuli were shown. The data points may be quantified by determining the number or proportion of data points where the subject was looking at the stimuli, from which it can be determined whether the attention level is as good as the predetermined acceptable attention level given the subject indications.

In some embodiments, the eye tracking data comprises two or more or all of the above types of data.

It is noted that in some embodiments where it can be assumed that the subject will have a sufficient level of attention during the entire process (for example communicative and collaborative patients), the methods disclosed herein may comprise determining if the calibration quality is higher than the predetermined minimum calibration quality and not the attention level. In other embodiments, it may be assumed that the calibration quality is sufficiently high without performing any checks on the calibration quality, and the method may comprise determining whether the attention level is higher than the predetermined attention level, and not the calibration quality.

It is noted that the calibration quality checks may include both determining whether the calibration quality itself is low (i.e. such that the accuracy of all data is low), and determining whether the position of the subject renders the eye tracking data of a lower accuracy (i.e. such that the data can be improved by repositioning the subject without the need for recalibration of the eye tracking device).

In some embodiments, the methods disclosed herein may comprise determining both whether the calibration quality is higher than the predetermined minimum calibration quality and whether the attention level is higher than the predetermined attention level. It is noted that the checks on calibration quality may be performed on the same or different eye tracking data for the same or different sequences of visual stimuli shown to the subject. For example, a first subset of eye tracking data may be check for calibration quality (for example eye tracking data obtained during a post-calibration sequence of visual stimuli) and a second subset of eye tracking data may be checked for attention level (for example eye tracking data obtained during an eye tracking-based test or task). Both calibration quality and attention level may be continuously checked by quantifying the data quality and/or attention level over a time window during the entire process of an eye tracking-based process with a subject. For example, both may be continuously monitored over a series of eye tracking-based tests or tasks to determine whether the calibration quality and/or attention level has degraded to an unacceptable level at any point, and providing a notification or pausing the process if either degrade to an unacceptable level. Advantageously, the method differentiates between low reliability data caused by poor calibration or poor positioning of the subject, and low reliability data caused by the attention level of the subject. As these causes are rectified in different manners, the overall reliability of the data can be improved by verifying both are above an acceptable level given the subject's indications or past performance in eye tracking-based tasks or tests.

Whilst the present disclosure primarily relates to determining the data quality for eye tracking-based tests and tasks relating to visual function or therapy, it will be appreciated that the methods and apparatuses disclosed herein may also be applied to other purposes and technical fields.

Having now described some illustrative embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

The apparatuses described herein may be embodied in other specific forms without departing from the characteristics thereof. The foregoing embodiments are illustrative rather than limiting of the described systems and methods. Scope of the apparatuses described herein is thus indicated by the appended claims, rather than the foregoing description, and changes that come within the meaning and range of equivalence of the claims are embraced therein.

## Claims

1. A computer-implemented method of validating eye tracking data for a system for performing eye tracking-based tests or tasks on a subject, comprising:
receiving eye tracking data of one or both eyes of a subject for each one of a sequence of visual stimuli, the eye tracking data indicative of one or more characteristics of the subject's gaze or position when each of the stimuli were displayed;
receiving one or more subject indications comprising at least one of the following: the subject's age; one or more pathological or physiological conditions of the subject; and data representative of a calibration quality and/or attention level for one or more previous eye tracking-based tests or tasks performed by the subject; and
one or more of the following steps:
a) quantifying a calibration quality of the eye tracking data and determining whether the calibration quality is lower than a predetermined minimum calibration quality associated with the subject indications, based at least on the eye tracking data the position of each visual stimuli and the subject indications; and/or
b) quantifying an attention level of the subject based on the eye tracking data, and determining whether the attention level of the subject is lower than a predetermined minimum attention level associated with the subject indications, based at least on the eye tracking data and the subject indications.

2. The method of claim 1, wherein the step of determining whether the calibration quality is lower than the minimum calibration quality and/or the step of determining whether the attention level is below the predetermined minimum attention level comprises the steps of:
- receiving or calculating, for one or both eyes, one or more statistical parameters describing a plurality of data points of the eye tracking data over time when each stimulus was shown, and/or one or more secondary parameters derived from said one or more statistical parameters;
- performing a comparison of the one or more statistical parameters or secondary parameters to one or more predetermined threshold values associated with the subject indications, the one or more threshold values indicative of acceptable calibration quality and/or attention level for the subject indications; and
- if a predetermined number of the one or more statistical parameters or secondary parameters do not meet the associated predetermined threshold value, determining that the calibration quality is lower than the predetermined minimum quality and/or the attention level is below the predetermined minimum attention level, and otherwise determining that the eye tracking data is acceptable.

3. The method of claim 1, wherein the step of determining whether the calibration quality is lower than the minimum calibration quality and/or the step of determining whether the attention level is below the predetermined minimum attention level comprises the step of inputting the eye tracking data and/or one or more statistical parameters describing the eye tracking data and/or one or more secondary parameters derived from one or more said statistical parameters, and the subject indications into an AI algorithm, wherein the AI algorithm is trained to determine whether the calibration quality and/or the attention level above or below a predetermined minimum calibration quality and/or attention level associated with the subject indications.

4. The method of any preceding claim, wherein the method includes step a) and the eye tracking data is obtained by an eye tracking device, and if it is determined that the eye tracking data is of a lower calibration quality than the predetermined minimum calibration quality, recommending or performing a recalibration of the eye tracking device and/or indicating that the subject should be repositioned to improve the calibration quality of the data.

5. The method of any preceding claim, wherein the method includes step b) and if it is determined that the eye tracking data is indicative of a lower attention level than the predetermined minimum attention level and/or it is determined the calibration quality is lower than the predetermined minimum calibration quality, obtaining new eye tracking data by:
displaying to the patient a second plurality of visual stimuli, the visual stimuli displayed individually in sequence, the visual stimuli having a plurality of spatial positions;
tracking, by an eye tracking device, the gaze of one or both of the eyes of the patient for each of the visual stimuli; and
generating, based on the tracked gaze, eye tracking data indicative of one or more characteristics of the subject's gaze or position during the display of each visual stimuli.

6. The method of claim 5, wherein the second plurality of visual stimuli have different static or dynamic visual characteristics or a smaller range of different static or dynamic visual characteristics compared to the visual stimuli used to generate the eye tracking data associated with an unacceptable attention level and/or calibration quality; and/or wherein the second plurality of visual stimuli are displayed over a smaller field of view than for the visual stimuli used to generate the eye tracking data of an unacceptable attention level and/or calibration quality; and/or wherein one or more audio stimuli are provided to the subject during the sequence of the second plurality of visual stimuli; and/or the visual stimuli are shown over a different time period compared to the visual stimuli used to generate the eye tracking data associated with an unacceptable attention level and/or calibration quality.

7. The method of any preceding claim, wherein the eye tracking data is eye tracking data obtained during calibration of an eye tracking device, or wherein the eye tracking data is eye tracking data obtained during an eye tracking-based test or task.

8. The method of any preceding claim, wherein the method comprises generating the eye tracking data by the following preceding steps:
displaying to a subject, a plurality of visual stimuli, the visual stimuli displayed individually in sequence;
tracking, by an eye tracking device, the gaze of one or both of the eyes of the subject for each of the visual stimuli; and
generating, based on the tracked gaze, eye tracking data indicative of one or more characteristics of the subject's gaze or position during the display of each visual stimuli.

9. The method of claim 8, wherein the eye tracking data excludes eye tracking data obtained within a predetermined amount of time after the initial appearance of one or more of the stimuli, and/or excludes eye tracking data obtained within a predetermined amount of time before the disappearance of one or more of the stimuli.

10. The method of claim 8 or 9, wherein the eye tracking data comprises data characterising the position of the gaze of one or both eyes, the method comprises determining the position of the gaze relative to the stimuli, and wherein one or more display characteristics of the visual stimuli change and/or audio stimuli is provided to the subject when it is determined that the subject is viewing the visual stimuli and/or when it is determined that the subject is not viewing the visual stimuli.

11. The method of any of claims 8 to 10, wherein the eye tracking data is continuously obtained and comprises data indicative of an attention level of the subject and/or calibration quality of the data, the method comprises quantifying the attention level of the subject and/or the calibration quality of the data, and wherein when it is determined that the subject is not paying attention and/or the calibration quality is below a predetermined minimum calibration quality, the sequence of visual stimuli is paused; optionally wherein the sequence of visual stimuli is resumed when it is determined from the continuously obtained data that the patient is paying attention and/or the calibration quality is above the predetermined minimum calibration quality.

12. The method of claim 11, wherein the sequence of visual stimuli is resumed when it is determined that the subject is paying attention and/or the calibration quality is above the predetermined minimum calibration quality, and the sequence is resumed by one of the following:
- displaying the same stimulus which was being displayed when it was determined that the subject was not paying attention; or
- discarding the data for the stimulus which was being displayed when it was determined that the subject was not paying attention and displaying an additional visual stimulus at the end of the sequence of visual stimuli;
- resetting the sequence of stimuli to a predetermined amount of time or a predetermined number of visual stimuli before the sequence was paused; or
- continuing the sequence of stimuli from the point where the process was paused and optionally discarding eye tracking data obtained while the process was paused, optionally further discarding eye tracking data obtained a predetermined amount of time before the process was paused and after the process was resumed.

13. The method of any of claims 8 to 12, wherein prior to generating the eye tracking data, the method comprises the steps of:
- tracking, via the eye tracking device or a device for tracking the subject, the position of the subject;
- indicating the position of the subject on a display device; and
- overlaying a subject positioning guide on the display device to indicate the correct position of the subject for obtaining the eye tracking data.

14. The method of any of claims 8 to 13, wherein the eye tracking device is configured to detect whether there are a plurality of pairs of eyes within the field of view of the eye tracking device, optionally wherein if a plurality of pairs are eyes are detected by the eye tracker, the eye tracking data is only generated for the lowermost pair of eyes being the subject's eyes.

15. An apparatus configured to perform the method according to any of claims 1 to 14, comprising:
- a display device configured to display the visual stimuli to the subject;
- an eye tracking device configured to track the gaze of the subject when the visual stimuli are displayed to the subject and to generate eye tracking data indicative of one or more characteristics of the subject's gaze or position; and
- one or more processors configured to control the display device to display the visual stimuli, to receive eye tracking data from the eye tracking device, and determine whether the eye tracking data is indicative of a lower quality than the predetermined minimum quality, based at least on the eye tracking data for each visual stimuli, and the one or more subject indications of the subject.

16. A computer program comprising computer-readable instructions which, when executed by a processor, cause the processor to execute a method according to any of claims 1 to 14.
